# EUROPÄISCHE PATENTSCHRIFT

(11) **EP 1 378 207 B1**
(45) Veröffentlichungstag und Bekanntmachung des Hinweises auf die Patenterteilung: **28.05.2008**
(21) Anmeldenummer: 03014961.1
(22) Anmeldetag: 01.07.2003
(51) Int. Cl.: A61C 1/14, A61B 17/16

(54) **Medizinisches oder dentalmedizinisches Handstück mit einer Drucktaste zum Lösen eines Werkzeugs**
Medical or dental handpiece with a push-button for releasing a tool
Pièce à main medicale ou dentaire avec un bouton-poussoir pour le désaccouplement d'un outil

(30) Priorität: 02.07.2002 DE 10229649
(43) Veröffentlichungstag der Anmeldung: 07.01.2004
(73) Patentinhaber: Kaltenbach & Voigt GmbH, 88400 Biberach / Riss (DE)
(72) Erfinder: Kuhn, Bernhard, 88400 Biberach (DE)
(74) Vertreter: Schmidt-Evers, Jürgen

(56) Entgegenhaltungen:
- EP-A- 0 374 276
- EP-A- 0 820 734
- DE-A- 2 905 484
- DE-A- 3 346 248

## Beschreibung

Die Erfindung bezieht sich auf ein medizinisches oder dentalmedizinisches Handstück nach dem Oberbegriff des Anspruchs 1.

Ein Handstück dieser Art ist z. B. in der DE-AS 1 092 607 beschrieben. Bei diesem vorbekannten Handstück weist eine im vorderen Endbereich des Handstücks angeordnete Haltevorrichtung für ein Werkzeug eine Antriebshülse auf, die quer zur Längsmittelachse des Handstücks angeordnet ist und in einem Drehlager mit zwei einen Abstand voneinander aufweisenden Wälzlagern drehbar gelagert ist. Die Antriebshülse bildet ein Einsteckloch mit einer seitlichen Einstecköffnung für das Werkzeug. Auf der der Einstecköffnung abgewandten Seite ist eine Drucktaste angeordnet, die durch Fingerdruck gegen eine elastische Rückstellkraft einschiebbar ist und dadurch ein Lösen des Werkzeugs bewirkt. Die Drucktaste weist die Querschnittsform eines Hutes auf, mit einem am unteren Rand der Hutform nach außen abstehenden Flansch, der eine Hinterschneidung in einem in das Handstück eingeschraubten Schraubteil hintergreift und dadurch unverlierbar am Handstück gehalten ist. Das Schraubteil dient zum einen einer axialen Begrenzung des Drehlagers und zum anderen der Führung und Halterung der Drucktaste. Bei dieser vorbekannten Ausgestaltung ist zwischen dem Schraubteil und dem Handstück eine nach außen auslaufende Schraubfuge vorhanden, die nicht nur das Aussehen des Handstücks herabsetzt, sondern in der sich auch Schmutz und Krankheitserreger ansammeln können. Außerdem bedarf es zum Ein -und Ausschrauben des Schraubteiles eines formschlüssig wirksamen Angriffselements am Schraubteil, das von außen zugänglich ist und somit ein weiteres Element bildet, in dem sich Schmutz und Krankheitserreger ansammeln können. Ferner wird auch durch ein solches Angriffselement das Aussehen des Handstücks herabgesetzt.

Bei einem Handstück moderner Bauweise ist das Schraubteil mit einem auf dem Rand der Montageöffnung aufliegenden Flansch ausgebildet, der der Führung und Halterung einer Drucktaste dient, die in vorbeschriebener Weise eine Hinterschneidung im Flansch hintergreift. Bei dieser Ausgestaltung ist zwischen dem Flansch und dem Handstück ebenfalls eine Schraubfuge vorhanden, wobei darüber hinaus der auf dem Gehäuse aufliegende Flansch zu einer vergrößerten Bauweise führt, was im Hinblick auf beengte Behandlungsstellen, insbesondere im Mundraum eines Patienten, vermieden werden soll.

Das Dokument EP 0820734 offenbart ein Handstück nach dem Oberbegriff des Anspruchs 1.

Der Erfindung liegt die Aufgabe zugrunde, bei einem Handstück der eingangs angegebenen Art die Verschmutzungsanfälligkeit zu vermindern.

Diese Aufgabe wird durch die Merkmale des Anspruchs 1 gelöst. Vorteilhafte Weiterbildungen der Erfindung sind in den Unteransprüchen beschrieben.

Bei der erfindungsgemäßen Ausgestaltung ist das Schraubteil wenigstens im Bereich seines das Gewinde aufweisenden Körperteils in der Montageöffnung versenkt angeordnet, wobei die Drucktaste mit geringem Führungsspiel in die Montageöffnung einfasst. Bei der erfindungsgemäßen Ausgestaltung ist deshalb nur die für die Bewegung der Drucktaste erforderliche Fuge vorhanden. Die Fuge zwischen dem Handstück und dem Einsatzteil ist verdeckt angeordnet und somit der Verschmutzung von außen entzogen. Dies gilt auch für ein Angriffselement zum Ein- und Ausschrauben eines Einsatzteils in Form eines Schraubteils, das ebenfalls verdeckt angeordnet ist und somit der Zugänglichkeit von Schmutz und Krankheitserregern entzogen ist. Es wird auch das Aussehen des Handstücks verbessert. Da das Einsatzteil in der Montageöffnung versenkt angeordnet ist, kann die erfindungsgemäße Ausgestaltung auch zu einer Verkleinerung der Bauweise führen.

In den Unteransprüchen sind Merkmale enthalten, die zu einfachen, kostengünstig herstellbaren und kleinen bzw. raumgünstigen Bauweisen beitragen. Weitere Weiterbildungen der Erfindung führen auch zu solchen Merkmalen, die eine einfache und schnelle Montage der Drucktaste ermöglichen.

Nachfolgend werden vorteilhafte Ausgestaltungen der Erfindung anhand von Zeichnungen näher erläutert. Es zeigt
- Fig. 1: ein Handinstrument zum Behandeln des menschlichen oder tierischen Körpers mit einem erfindungsgemäßen Handstück in der Seitenansicht;
- Fig. 2: den vorderen Bereich des Handstücks nach Fig. 1 im vertikalen Längsschnitt.

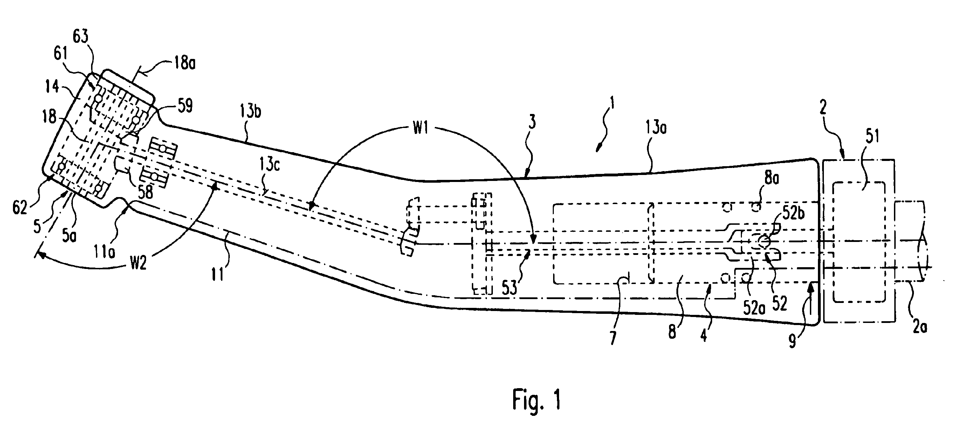

Das in Fig. 1 in seiner Gesamtheit mit 1 bezeichnete Behandlungsinstrument besteht aus einem hinteren Instrumententeil, nämlich einem sogenannten Anschlussteil 2, und einem vorderen Instrumententeil, nämlich dem sogenannten Handstück 3, die durch eine Kupplung 4 insbesondere eine Steckkupplung, vorzugsweise eine Steck/Dreh-Kupplung, lösbar miteinander verbunden sind. Beim vorliegenden Ausführungsbeispiel ist am vorderen Ende des Behandlungsinstruments 1 eine Haltevorrichtung 5 mit einer seitlichen Einstecköffnung 5a für ein Werkzeug angeordnet, wobei das Werkzeug seitlich absteht. Das längliche Handstück 3 in Form eines Schaftes kann sich gerade (nicht dargestellt) oder zur dem Werkzeug abgewandten Seite hin gekrümmt (nicht dargestellt) oder mit zwei einen stumpfen Winkel W1 einschließenden Schaftabschnitten 13a, 13b winkelförmig erstrecken. Die Steck/Dreh-Kupplung wird durch eine im Querschnitt runde Kupplungsausnehmung 7 und einen darin mit geringem Bewegungsspiel einsteckbaren Kupplungszapfen 8 gebildet. Beim vorliegenden Ausführungsbeispiel ist die Kupplungsausnehmung 7 am hinteren Ende des Handstücks 3 angeordnet, und der im wesentlichen zylindrische Kupplungszapfen 8 erstreckt sich vom Anschlussteil 2 nach vorne. Im gekuppelten Zustand sind die Kupplungsausnehmung 7 und der Kupplungszapfen 8 durch eine Verrastungsvorrichtung lösbar miteinander verrastet. Diese weist ein Verrastungselement 9 auf, das in dem einen Kupplungsteil radial beweglich gelagert und durch eine Federkraft in eine die Teilungsfuge durchsetzende Verrastungsstellung vorgespannt ist, in der das Verrastungselement 9 in eine Ringnut im anderen Kupplungsteil einfasst. Eine solche Verrastungsvorrichtung verrastet beim Kuppeln selbsttätig, und sie ist beim Entkuppeln durch eine manuelle axiale Zugkraftausübung überdrückbar, wobei das Verrastungselement 9 selbsttätig in seine Freigabestellung verdrängt wird.

Das Anschlussteil 2 ist mit einer flexiblen Versorgungsleitung 2a verbunden, die mit einem nicht dargestellten Steuergerät verbunden ist. Das Handstück 3 ist auf dem Kupplungszapfen 8 frei drehbar gelagert, wodurch die Handhabbarkeit verbessert wird. Durch die Steck/Dreh-Kupplung 4 erstreckt sich wenigstens eine Medienleitung 11 für ein Behandlungs- oder Antriebsmedium, z. B. Wasser, Druckluft oder ein Wasser/Luft-Gemisch (Spray). Die Medienleitung 11 erstreckt sich Z-förmig durch die hohlzylindrische Teilungsfuge zwischen der Kupplungsausnehmung 7 und dem Kupplungszapfen 8, wobei die Medienleitung 11 die Teilungsfuge im Bereich einer Ringnut im Kupplungszapfen 8 oder in der Kupplungsausnehmung 7 durchsetzt, so dass in jeder Drehstellung der Mediendurchgang gewährleistet ist. Beiderseits vom Durchgang durch die Teilungsfuge ist diese durch einen Dichtring 8a abgedichtet, der in einer Ringnut in der Wandung der Kupplungsausnehmung 7 oder in der Mantelfläche des Kupplungszapfens 8 angeordnet sein kann. Hierdurch ist eine freie Drehbarkeit um 360 ° und mehr gewährleistet. Die Medienleitung 11 erstreckt sich vom hinteren Ende des Behandlungsinstruments 1 zu dessen vorderem Endbereich, wobei sie teilweise als Kanal im Instrumentenkörper oder als Schlauch- bzw. Rohrleitung verlaufen kann. Die Medienleitung 11 mündet im vorderen Endbereich des Behandlungsinstruments 1 aus diesem aus, wobei diese Mündungsöffnung 11a auf die Behandlungsstelle bzw. auf die Spitze des Werkzeugs gerichtet ist.

Am vorderen Ende des Handstücks 3 befindet sich ein verdickter Kopf 14, in dem eine Aufnahmehülse 18 um eine quer verlaufende Drehachse 18a drehbar gelagert ist, die an einer Seite die Einstecköffnung 5a bildet und in die das Werkzeug mit seinem Schaft einsteckbar und in an sich bekannter Weise durch eine Fixiervorrichtung lösbar fixierbar ist.

Der zwischen der Mittelachse 13c des vorderen Schaftabschnitts 13b und der Drehachse 18a der Aufnahmehülse 18 eingeschlossene Winkel W2 beträgt vorzugsweise mehr als 90°, insbesondere im Wesentlichen 100°. Eine solche Ausgestaltung ist günstig und unter Berücksichtigung der Anatomie im Mundraum eines Patienten besonders günstig.

Ein Antriebsmotor 51, z. B. ein Elektromotor, ist im strichpunktiert ergänzten Anschlussteil 2 angeordnet und durch eine im Schaft des Handstücks 3 drehbar gelagerte Antriebswelle oder einen Antriebswellenzug 53 mit einem oder mehreren Antriebswellenabschnitten mit der Aufnahmehülse 18 antriebsmäßig verbunden. Im Bereich der Steckkupplung 4 weist die Antriebswelle 53 eine Steckkupplung 52 mit zwei formschlüssig miteinander korrespondierenden Steckkupplungselementen 52a, 52b auf, wodurch beim Kuppeln und Entkuppeln der Steckkupplung 4 zugleich ein Kuppeln und Entkuppeln der Steckkupplung 52 möglich ist.

Die Antriebsverbindung zwischen der Antriebswelle 53 und der Aufnahmehülse 18 ist durch ein Zahnradgetriebe oder Winkelzahnradgetriebe mit einem Zahnrad 58 oder Kegelzahnrad am vorderen Ende der Antriebswelle 53 und einem Zahnrad 59 oder Kegelzahnrad auf der Aufnahmehülse 18 gebildet. Der Zahneingriff zwischen den Zahnrädern 58, 59 ist bezüglich der Antriebswelle 53 auf deren dem Werkzeug abgewandten Seite angeordnet. Die Aufnahmehülse 18 kann mittels zwei Wälzlagern 61, 62 im Kopf 14 drehbar gelagert sein, die einen längs der Drehachse 18a gerichteten Abstand voneinander aufweisen, der größer ist als das Zahnrad 58, so dass letzteres mit dem Zahnrad 59 dazwischen angeordnet werden kann, einschließlich dem Zahnrad 59, das auf der der Werkzeugöffnung 5a abgewandten Seite des Zahnrades 58 und zugleich auf der der Werkzeugöffnung 5a zugewandten Seite des Wälzlagers 61 angeordnet ist, das von der Werkzeugöffnung 5a weiter entfernt angeordnet ist als das andere Wälzlager 62. Die Lageranordnung mit den Wälzlagern 61, 62 ist durch eine Montageöffnung 63 montierbar bzw. demontierbar, die im Kopf 14 auf dessen der Werkzeugöffnung 5a abgewandten Seite angeordnet ist.

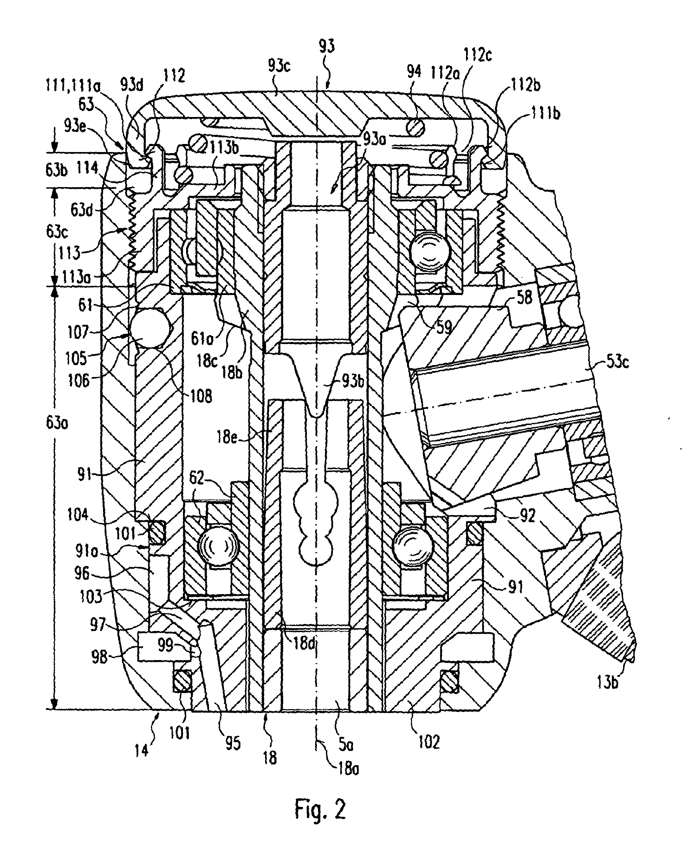

Wie Fig. 2 beispielhaft zeigt, kann die Aufnahmehülse 18 im Kopf 14 in einer Lagerhülse 91 gelagert sein, die in die Montageöffnung 63 drehfest eingesetzt ist. Die Montageöffnung 63 weist in ihrem vorderen Bereich eine gegebenenfalls konvergent gestufte Passung 91a auf, in der die Lagerhülse 91 für die Wälzlager 61, 62 sitzt. Der freie Endbereich der Montageöffnung 63 ist als zylindrischer Führungsabschnitt 63b ausgebildet. Dazwischen ist ein Schraubabschnitt 63c mit einem Innengewinde angeordnet, dessen Durchmesser gleich oder kleiner ist als der Durchmesser des Führungsabschnitts 63b, wie es Fig. 2 zeigt.

Beim in Fig. 2 dargestellten Ausführungsbeispiel sitzen die Außenringe der Wälzlager 61, 62 jeweils in einer Passung in der Lagerhülse 91, wobei sie an einer Schulterfläche anliegen können, die der Werkzeugöffnung 5a abgewandt ist.

Es ist möglich und aus Gründen einfacher Bauweise und guter Laufeigenschaften vorteilhaft, die Lagerhülse 91 als gemeinsamen Außenring für beide Wälzlager auszubilden, so dass sich ein zweireihiges Wälzlager ergibt. Bei einer solchen Ausgestaltung entfallen die Außenringe der Wälzlager, wobei die zugehörigen Laufrillen in der Innenmantelfläche der Lagerhülse 91 angeordnet sind.

Es ist bei allen vorbeschriebenen Ausgestaltungen möglich und vorteilhaft, wenn die Lagerhülse 91 sich im Wesentlichen bis zu der die Einstecköffnung 5a enthaltenen Seite des Kopfes 14 erstreckt und im dieser Seite zugewandten Endbereich der Lagerhülse 91 wenigstens ein Zuführungskanalabschnitt 95 für ein Behandlungsmedium, z. B. Druckluft und/oder Wasser und/oder ein Wasser/Luftgemisch (Spray) angeordnet ist, dessen Ausmündung in Richtung auf die Behandlungsstelle bzw. auf das Werkzeug gerichtet ist. Zur Verbindung des konvergenten Zuführungsleitungsabschnitts 95 mit der Medienleitung 11, die beim Ausführungsbeispiel nach Fig. 2 nicht bei 11a ausmündet, kann ein Ringkanal 96 in der Außenmantelfläche der Lagerhülse 91 angeordnet sein, der sich über den gesamten Umfang oder nur über einen Teil des Umfangs erstreckt, und in dessen rückseitigen Bereich der Medienleitung 11 mündet. Der Ringkanal 96 kann durch einen schräg verlaufenden Verbindungskanal 97 mit dem Zuführungsleitungsabschnitt 95 verbunden sein.

Beim Vorhandensein von zwei Medienleitungen 11 für zwei unterschiedliche Behandlungsmedien, z. B. Luft und Wasser, kann ein zweiter, axial versetzt angeordneter Ringkanal 98 vorgesehen sein, der durch einen quer verlaufenden Verbindungskanal 99 ebenfalls mit dem Zuführungsleitungsabschnitt 95 verbunden ist. Zu beiden Seiten des oder der Ringkanäle 96, 98 ist die Lagerhülse 91 in der Passung 91a bzw. Aufnahmebohrung jeweils durch einen Dichtring 101 abgedichtet, der in einer Ringnut in der Außenmantelfläche der Führungshülse 91 oder in der Innenmantelfläche der Passung 91a sitzt und mit der jeweils gegenüberliegenden Wandung abdichtend zusammenwirkt.

Es können mehrere Zuführungskanalabschnitte 95 auf dem Umfang verteilt angeordnet sein, die jeweils mit dem oder den zugehörigen Ringkanälen 96, 98 verbunden sind.

Beim Ausführungsbeispiel ist die Lagerhülse 91 in dem Bereich, in dem der wenigstens eine Zuführungsleitungsabschnitt 95 angeordnet ist, radial verdickt, wobei sie sich radial einwärts bei Wahrung eines Bewegungsspiels bis zur Antriebshülse 18 erstrecken kann. Durch diese Verdickung 102 lässt sich eine Innenschulter 103 bilden, an der das Wälzlager 62 anliegt, hier mit seinem Außenring.

Zur axialen Begrenzung der Lagerhülse 91 zur Einstecköffnung 5a hin weist die Passung 91a bzw. das Aufnahmeloch wenigstens eine Innenschulter 104 auf, an der die Lagerhülse 91 mit einer Außenschulter anliegt, vorzugsweise in einer axialen Position, in der die Einstecköffnung 5a mit der zugehörigen Seite des Kopfes 14 abschließt.

Die Lagerhülse 91 ist durch eine Drehsicherung 105 gegen Drehen gesichert, Hierzu weist sie einen von ihrer Mantelfläche abstehenden Sicherungsvorsprung 106 auf, der in eine axiale Sicherungsnut 107 in der Innenmantelfläche des Aufnahmelochs einfasst, wobei die Nut 107 zur der Einstecköffnung 5a abgewandten Seite hin ausläuft. Der Sicherungsvorsprung 106 ist vorzugsweise durch eine Kugel gebildet, die in ein Loch 108 eingesetzt und arretiert ist, z. B. durch Verstemmen.

Der vordere Antriebswellenabschnitt 53c durchgreift mit seinem vorderen Zahnrad 58 die Lagerhülse 91 in einem radialen Durchführungsloch 92, und es kämmt mit dem teilweise innerhalb der Lagerhülse 91 und zwischen den Innenlagerringen angeordneten Zahnrad 59. Letzteres ist bezüglich dem Zahnrad 58 vorzugsweise zur der Einstecköffnung 5a abgewandten Seite versetzt angeordnet, so dass es mit dem Zahnrad 58 auf dessen der Einstecköffnung 5a abgewandten Seite kämmt. Beim Ausführungsbeispiel ist das Zahnrad 59 an einer endseitigen Verdickung 18b der Aufnahmehülse 18 angeordnet, z. B. einteilig ausgebildet. Auf der wenigstens abschnittsweise außen zylindrisch ausgebildeten Verdickung 18b kann der Innenlagerring 61b der zugehörigen Wälzkörperreihe gelagert sein und vorzugsweise mit seinem der Einstecköffnung 5a zugewandten Ende an einem Flansch 18c der Verdickung 18b anliegen, an dem auch die Verzahnung auf der dem Innenlagerring 61b abgewandten Seite ausgebildet sein kann. Zwischen der Drehachse 18a und der Mittelachse 13c ist der Winkel W2 eingeschlossen.

In der Aufnahmehülse 18 kann in deren der Einstecköffnung 5a zugewandten Bereich eine an sich bekannte Klemmhülse 18d mit einander gegenüberliegenden Klemmbacken 18e für den Schaft des Werkzeugs 5 angeordnet sein. Zum Lösen des Werkzeugs 5 ist auf der der Einstecköffnung 5a abgewandten Seite eine Lösevorrichtung 93 angeordnet, mit einem Lösestift 93a, der in der Aufnahmehülse 18 axial verschiebbar gelagert ist und zum Lösen des Werkzeugs mit einem Keil 93b zwischen die Klemmbacken 18e schiebbar ist. Zum Betätigen des Lösestiftes 93a ist eine Drucktaste 93c vorgesehen, die durch Fingerdruck gegen die Kraft einer durch eine Deckelwand der Drucktaste 93c verdeckt angeordneten Druckfeder 94 aus einer in Fig. 2 dargestellten Ausgangsstellung in eine nicht dargestellte, in den Kopf 14 eingeschobene Lösestellung verschiebbar ist. Die Rückstellung des Lösestiftes 93a und der Drucktaste 93c erfolgen selbsttätig durch die Federkraft.

Die Außenabmessung der Drucktaste 93c ist mit einem geringen Bewegungs- oder Führungsspiel an die Querschnittsgröße des Führungsabschnitts 63b angepasst. Die Querschnittsform der Drucktaste 93c ist mit der Deckelwand und einer von dieser in Richtung auf den Kopf 14 abstehenden Ringwand 93d hutförmig. In der Ausgangsstellung greift die Ringwand 93d wenigstens geringfügig in den Führungsabschnitt 63b ein. Zur Vereinfachung der Einführung bei der Montage kann die Ringwand 93d an ihrem freien Außenrand eine gerundete oder schräge Einführungsfläche 93e aufweisen, die sich in der Ausgangsstellung der Drucktaste 93 innerhalb des Führungsabschnitts 63b befindet, so dass der Ringspalt zwischen der Ringwand 93d und der Innenmantelfläche des Führungsabschnitts 63b auf das Führungsspiel beschränkt ist.

Die Auswärtsbewegung der Drucktaste 93 ist durch einen oder mehrere auf einem Umfang der Drucktaste 93 verteilt angeordnete Anschläge 111 begrenzt, die formschlüssig mit einem oder mehreren auf einem Umfang verteilt angeordneten Gegenanschlägen 112 zusammenwirken und den oder die Gegenanschläge 112 hintergreifen. Der oder die Gegenanschläge 112 sind an einem Einsatzring 113 angeordnet, der in der Montageöffnung 63 soweit versenkt angeordnet ist, dass der Führungsabschnitt 63b einen Ringfreiraum 63d umgibt. Beim Ausführungsbeispiel ist der Einsatzring 113 ein Schraubring mit einem Außengewinde, das in den Schraubabschnitt 63c eingeschraubt ist. Der Einsatzring 113 kann mit einer Umfangswand 113a und einer sich radial nach innen erstreckenden Flanschwand 113b im Querschnitt winkelförmig ausgebildet sein. Zur Verringerung der axialen Länge kann die Umfangswand 113a in eine entsprechende Ringausnehmung am betreffenden Ende der Lagerhülse 91 einfassen, so dass die Umfangswand 113a das zugehörige Wälzlager 61 z. B. mit radialem Abstand umgibt. Hierbei kann die Flanschwand 113b die Lagerhülse 91 und/oder das Wälzlager 61 auf der der Einstecköffnung 5a abgewandten Seite axial begrenzen.

Die Anschläge 111 oder die Gegenanschläge 112 sind derart elastisch nachgiebig, dass die so gebildete Anschlagvorrichtung die Drucktaste gegen die Kraft der Feder 94 bis zu einer bestimmten Größe der Federkraft axial positioniert. Wenn die Federkraft den bestimmten Wert übersteigt, geben die elastisch nachgiebigen Anschlagteile nach, so dass die Anschlagvorrichtung überdrückt werden kann. Dies gilt sowohl für eine nach außen gerichtete Axialbewegung als auch für eine nach innen gerichtete Axialbewegung der Drucktaste 93. Hierdurch ist die Montage oder Demontage der Drucktaste gewährleistet. Für eine Montage wird die Drucktaste 93 mit ihren Anschlägen 111 über die Gegenanschläge 112 geschoben, wobei die elastisch nachgiebigen Anschlagteile ausweichen und in der Ausgangsstellung der Drucktaste 93 zurückfedern, wodurch der Hintergriff gewährleistet ist.

Beim Ausführungsbeispiel ist ein Ringanschlag 111 als Ringwulst 111a an der Innenumfangswand der Ringwand 93d vorgesehen. Die Gegenanschläge 112 sind an sich im Wesentlichen axial erstreckenden Federarmen 114 angeordnet, die sich vom Einsatzring 113, insbesondere von dessen Flanschwand 113b zur der Einstecköffnung 5a abgewandten Seite hin erstrecken und an ihren freien Endbereichen die nach außen vorstehenden Gegenanschläge 112 in Form von Ringwulstsegmenten 112a aufweisen.

Um das vorbeschriebene Überdrücken der Anschlagvorrichtung wenigstens für eine einwärts gerichtete Montagebewegung und vorzugsweise auch für eine nach außen gerichtete Demontagebewegung zu gewährleisten, weisen der oder die Anschläge 111 und/oder der oder die Gegenanschläge 112 durch schräge oder gerundete Flanken gebildete Anlaufflächen 111b, 112b auf, die das elastische Ausweichen bzw. Ausbiegen der elastisch nachgiebigen Anschlagteile gewährleisten.

Innerhalb der Federarme 114 ist die Druckfeder 94 angeordnet, wobei die Federarme 114 eine Zentrierung für die Druckfeder 94 bilden können. Wie Fig. 2 zeigt, können die Wände der Druckfeder 94 aufgrund ihrer konvergenten bzw. divergenten Form in eine wenigstens teilweise nebeneinander angeordnete Position zusammengedrückt werden, wodurch die Druckfeder 94 einen geringen axialen Bauraum benötigt.

Die Haupt-Teile des Kopfes 14 des Handstücks 3, nämlich die Antriebshülse 18, die Lagerhülse 91, die Umfangswand 14a des Kopfes, die Drucktaste 93 und der Einsatzring 114 bestehen vorzugsweise aus korrosionsfestem Stahl.

Der Einsatzring 113 weist von außen zugängliche Angriffselemente auf, mit denen ein Drehwerkzeug zum Ein- oder Ausschrauben des Einsatzrings 113 zusammenwirken kann. Beim Ausführungsbeispiel können die Angriffselemente durch die zwischen den Ringsegmenten angeordneten Lücken 112c gebildet sein.

## Patentansprüche

1. Medizinisches oder dentalmedizinisches Handstück (3), das in seinem vorderen Endbereich eine seitliche Einstecköffnung (5a) für ein Werkzeug aufweist und der Einstecköffnung (5a) gegenüberliegend eine Montageöffnung (63) mit einem darin einsetzbaren Einsatzteil (113) sowie eine Drucktaste (93c) aufweist, die zwischen einer äußeren Ausgangsstellung und einer inneren Lösestellung verschiebbar gelagert ist und durch eine Federkraft in ihre Ausgangsstellung vorgespannt ist, in der die Drucktaste (93c) mit wenigstens einem an ihr angeordneten Anschlag (111) an wenigstens einem an Einsatzteil (113) vorgesehenen Gegenanschlag (112) abgestützt ist,
**dadurch gekennzeichnet,**
**dass** das Einsatzteil (113) in der Montageöffnung (63) so weit versenkt angeordnet ist, dass im freien Randbereich der Montageöffnung (63) ein hohlzylindrischer Führungsabschnitt (63b) verbleibt, und dass die Drucktaste (93c) mit geringem Bewegungsspiel an die Querschnittsgröße des Führungsabschnitts (63b) angepasst ist und darin geführt wird.

2. Handstück nach Anspruch 1,
**dadurch gekennzeichnet,**
**dass** der Anschlag (111) und/oder der Gegenanschlag (112) quer zur Schieberrichtung der Drucktaste (93) elastisch nachgiebig sind, so dass sie dann, wenn die Drucktaste (93) mit einer die erforderliche Anschlagkraft übersteigenden Schubkraft ein- oder auch ausgeschoben wird, der Anschlag (111) und/oder Gegenanschlag (112) elastisch ausweicht und in der Ausgangsstellung selbsttätig zurückfedert.

3. Handstück nach Anspruch 1 oder 2,
**dadurch gekennzeichnet,**
**dass** der Anschlag (111) an der Innenseite einer sich nach innen erstreckenden Ringwand (93d) der Drucktaste (93c) angeordnet ist.

4. Handstück nach Anspruch 3,
**dadurch gekennzeichnet,**
**dass** der Anschlag (111) durch eine Ringwulst (111a) an der Ring wand (93d) gebildet ist.

5. Handstück nach einem der vorherigen Ansprüche,
**dadurch gekennzeichnet,**
**dass** mehrere Gegenanschläge (112) vorgesehen sind, die segmentförmig auf dem Umfang verteilt angeordnet sind.

6. Handstück nach Anspruch 5,
**dadurch gekennzeichnet,**
**dass** die Gegenanschläge (112) an mehreren auf dem Umfang verteilt angeordneten Federarmen (114) angeordnet sind, die vom Einsatzteil etwa axial zur der Einstecköffnung (5a) abgewandten Seiten hin abstehen.

7. Handstück nach einem der vorherigen Ansprüche,
**dadurch gekennzeichnet,**
**dass** der Anschlag (111) und/oder der Gegenanschlag (112) innenseitig und/oder außenseitig schräg oder gerundete Einführungsflächen (111b, 112b) aufweist.

8. Handstück nach Anspruch 6 oder 7,
**dadurch gekennzeichnet,**
**dass** die Federarme (114) sich etwa achsenparallel erstrecken.

9. Handstück nach einem der Ansprüche 3 bis 8,
**dadurch gekennzeichnet,**
**dass** die Drucktaste (93) eine hutförmige Querschnittsform hat und die Ringwand (93d) durch die Umfangswand der Drucktaste (93) gebildet ist.

10. Handstück nach einem der vorherigen Ansprüche,
**dadurch gekennzeichnet,**
**dass** das Einsatzteil (113) ein Schraubteil ist.

11. Handstück nach Anspruch 10,
**dadurch gekennzeichnet,**
**dass** zwischen der Drucktaste (93c) und dem Schraubteil (95) eine Druckfeder (94) angeordnet ist.

12. Handstück nach Anspruch 11,
**dadurch gekennzeichnet,**
**dass** die Druckfeder (94) hohlkegelförmig geformt ist.

## Claims

1. Medical or dental-medical handpiece (3) which has in its forward end region a lateral insertion opening (5a) for a tool and, lying opposite the insertion opening (5a), an assembly opening (63) with an insert part (113) insertable therein and the push-button (93c) which is mounted displaceably between an outer initial position and an inner release position and is biased by a spring force into its initial position, in which the push-button (93c) is supported with at least one stop (111) arranged thereon on at least one counter-stop (112) provided on the insert part (113),
**characterised in that**,
the insert part (113) is arranged sunk so far into the assembly opening (63) that in the free edge region of the assembly opening (63) a hollow cylindrical guide section (63b) remains, and **in that** the push-button (93c) is adapted, with a slight play for movement, to the cross-sectional size of the guide section (63b) and is guided therein.

2. Handpiece according to claim 1,
**characterised in that**,
the stop (111) and/or the counter-stop (112) are elastically yielding transverse to the displacement direction of the push-button (93), so that the push-button, when the push-button (93) is displaced inwardly or outwardly with a force exceeding the required stop force, elastically evades the stop (111) and/or counter-stop (112) and self-actingly springs back into the initial position.

3. Handpiece according to claim 1 or 2,
**characterised in that**,
the stop (111) is arranged on the inside of an inwardly extending ring wall (93d) of the push-button (93c).

4. Handpiece according to claim 3,
**characterised in that**,
the stop (111) is formed by a ring bulge (111a) on the ring wall (93d).

5. Handpiece according to any preceding claim,
**characterised in that**,
a plurality of counter-stops (112) are provided which are arranged segment-like distributed on the perimeter.

6. Handpiece according to claim 5,
**characterised in that**,
the counter-stops (112) are arranged on a plurality of spring arms (114) arranged distributed on the perimeter, which arms project from the insert part approximately axially to the sides away from the insertion opening (5a).

7. Handpiece according to any preceding claim,
**characterised in that**
the stop (111) and/or the counter-stop (112) has on the inside and/or on the outside oblique or rounded introduction surfaces (111b, 112b).

8. Handpiece according to claim 6 or 7,
**characterised in that**,
the spring arms (114) extend approximately axis parallel.

9. Handpiece according to any of claims 3 to 8,
**characterised in that**,
the push-button (93) has a hat-like cross-sectional form and the ring wall (93d) is formed by the peripheral wall of the push-button (93).

10. Handpiece according to any preceding claim,
**characterised in that**,
the insert part (113) is a screw part.

11. Handpiece according to claim 10,
**characterised in that**,
a compression spring (94) is arranged between the push-button (93c) and the screw part (95).

12. Handpiece according to claim 11,
**characterised in that**,
the compression spring (94) is hollow conical shaped.

## Revendications

1. Pièce à main (3) médicale ou de médecine dentaire, qui présente dans sa zone d'extrémité avant une ouverture d'emboîtement (5a) latérale pour un outil et en face de l'ouverture d'emboîtement (5a) une ouverture de montage (63) avec un insert (113) pouvant être inséré à l'intérieur et une touche de pression (93c), qui est montée de façon à pouvoir être déplacée entre une position initiale extérieure et une position de desserrage intérieure et est prétendue par une force de ressort dans sa position initiale, dans laquelle la touche de pression (93c) est soutenue avec au moins une butée (111) disposée sur la touche sur au moins une contre-butée (112) prévue sur l'insert (113),
**caractérisée en ce que**
l'insert (113) est disposé noyé dans l'ouverture de montage (63) suffisamment pour que, dans la zone périphérique libre de l'ouverture de montage (63), il reste une partie de guidage (63b) cylindrique creuse, et **en ce que** la touche de pression (93c) est adaptée avec un faible jeu de mouvement à la grandeur de section de la partie de guidage (63b) et est guidée à l'intérieur.

2. Pièce à main selon la revendication 1,
**caractérisée en ce que**
la butée (111) et/ou la contre-butée (112) sont élastiquement souples transversalement à la direction de coulissement de la touche de pression (93), de sorte que, dans le cas où la touche de pression (93) est introduite ou sortie par glissement avec une force de poussée dépassant la force de butée nécessaire, la butée (111) et/ou la contre-butée (112) se déforme élastiquement et revient automatiquement à la façon d'un ressort dans la position initiale.

3. Pièce à main selon la revendication 1 ou 2,
**caractérisée en ce que**
la butée (111) est disposée sur le côté intérieur d'une paroi annulaire (93d), s'étendant vers l'intérieur, de la touche de pression (93c).

4. Pièce à main selon la revendication 3,
**caractérisée en ce que**
la butée (111) est formée par un gonflement annulaire (111 a) sur la paroi annulaire (93d).

5. Pièce à main selon l'une quelconque des revendications précédentes,
**caractérisée en ce que**
plusieurs contre-butées (112) sont prévues, lesquelles sont disposées avec une répartition en forme de segment sur le pourtour.

6. Pièce à main selon la revendication 5,
**caractérisée en ce que**
les contre-butées (112) sont disposées sur plusieurs bras à ressort (114) répartis sur le pourtour, qui débordent de l'insert à peu près axialement par rapport aux côtés opposés à l'ouverture d'emboîtement (5a).

7. Pièce à main selon l'une quelconque des revendications précédentes,
**caractérisée en ce que**
la butée (111) et/ou la contre-butée (112) présente(nt) des surfaces d'introduction (111 b, 112b) en biais inclinées ou arrondies côté intérieur et/ou extérieur.

8. Pièce à main selon la revendication 6 ou 7,
**caractérisée en ce que**
les bras à ressort (114) s'étendent sur des axes à peu près parallèles.

9. Pièce à main selon l'une quelconque des revendications 3 à 8,
**caractérisée en ce que**
la touche de pression (93) a une forme de section en forme de chapeau et la paroi annulaire (93d) est formée par la paroi périphérique de la touche de pression (93).

10. Pièce à main selon l'une quelconque des revendications précédentes,
**caractérisée en ce que**
l'insert (113) est une pièce vissée.

11. Pièce à main selon la revendication 10,
**caractérisé en ce que**
un ressort de pression (94) est disposé entre la touche de pression (93c) et la pièce vissée (95).

12. Pièce à main selon la revendication 11,
**caractérisée en ce que**
le ressort de pression (94) est formé en forme de cône creux.
